Europäisches Patentamt

European Patent Office

Office européen des brevets

(11) Numéro de publication: **0 343 600**

**A1**

## (12) DEMANDE DE BREVET EUROPEEN

(21) Numéro de dépôt: 89109274.4

(51) Int. Cl.⁴: **A61B 6/00**

(22) Date de dépôt: 23.05.89

(30) Priorité: 24.05.88 IT 2070588

(43) Date de publication de la demande:
29.11.89 Bulletin 89/48

(84) Etats contractants désignés:
AT BE CH DE ES GB GR LI LU NL SE

(71) Demandeur: **GENERAL MEDICAL MERATE S.p.A.**
**Via Sabotino 38/b**
**I-24068 Seriate (Bergamo)(IT)**

Demandeur: **Azancot, Isaac**
**9 Rue Barbette**
**F-25003 Paris(FR)**

(72) Inventeur: **Azancot, Isaac**
**9 Rue Barbette**
**F-25003 Paris(FR)**

(74) Mandataire: **Dragotti, Gianfranco**
**SAIC BREVETTI s.r.l. Viale Bianca Maria, 15**
**I-20122 Milano(IT)**

(54) **Procédé de calibration numérique d'une installation de radiologie et installation de calibration numérique pour la mise en oeuvre du procédé.**

(57) Procédé de calibration numérique d'une installation de radiologie, comprenant une table (1), un arceau (3) supportant à ses deux extrémités des moyens d'émission (4) d'un faisceau de rayons X et des moyens de réception dudit faisceau (5), des moyens (7,8) de stockage, de traitement et de visualisation des images, et des moyens (9) pour commander les déplacements dans ladite installation. Une grille (10) calibrée, sert à évaluer les paramètres relatifs aux coefficients d'agrandissement et de distorsion de l'image réalisée. Lesdits paramètres sont transférés à un ordinateur (8) d'analyse d'images et à un ordinateur (9) commandant le positionnement de l'installation.

FIG.1

## PROCEDE DE CALIBRATION NUMERIQUE D'UNE INSTALLATION DE RADIOLOGIE ET INSTALLATION DE CALIBRATION NUMERIQUE POUR LA MISE EN OEUVRE DU PROCEDE

L'invention concerne un procédé de calibration numérique d'une installation de radiologie, plus spécialement destinée à des procédures d'angiographie et d'angioplastie et l'installation pour le mise en oeuvre de ce procédé.

On connaît déjà une installation de radiologie comprenant une table de position générale au moins sensiblement horizontale réglable et blocable, sur laquelle peut reposer le corps d'un patient à examiner ; un arceau, notamment isocentrique, supportant à ses deux extrémités des moyens d'émission d'un faisceau de rayons X (à l'extrémité inférieure au dessous de la table) et des moyens de réception du dit faisceau (à l'extrémité supérieure au dessus de la table et connus par l'homme du métier sous le nom d'amplificateur) également réglable et blocable; des moyens de stockage, traitement ; visualisation des images associés aux moyens de réception, notamment par analyse numérique des images produites, grâce en particulier à un ordinateur. Des moyens supports de la table et de l'arceau sont déplaçables et blocables grâce à des moyens d'entraînement et des moyens de commande.

Avec une telle installation connue, on peut placer le corps d'un patient à examiner sur la table et régler la position de la table et/ou de l'arceau pour une certaine position relative correspondant à certains écartements et certains angles d'incidence et cela, selon les nécessités imposées par l'examen souhaité.

Plusieurs développements ont été apportés récemment à ces installations. Tout d'abord la technique de numérisation des images a facilité la mise en oeuvre de l'installation en ce qui concerne le traitement de l'image. Ensuite, on a proposé d'automatiser les déplacements de la table et de l'arceau grâce à un ordinateur de commande (voir document FR 2 588 745).

Par ailleurs récemment, se sont développées les procédures d'angioplastie consistant à repérer d'abord, sous contrôle angiographique, l'existence d'une sténose chez le patient puis à introduire dans l'artère à l'endroit de cette sténose un ballon de calibre approprié dont le gonflement, répété si nécessaire plusieurs fois, permet d'augmenter le calibre de l'artère.

La bonne exécution de ces procédures d'angioplastie nécessiterait la connaissance rapide et précise des diamètres internes ou calibres de l'artère dans une portion saine et à l'endroit d'une sténose. En effet, en particulier, la taille du ballon à utiliser est fonction de ces diamètres internes.

Or, les installations de radiologie actuellement utilisées couramment, ne permettent qu'une appréciation relative des dimensions mais non leur connaissance absolue lors d'une lecture directe de l'image. Et cette connaissance des dimensions en valeur absolue est d'autant plus importante que les positions relatives table-arceau sont en nombre infini.

L'invention a donc pour objet essentiel de permettre d'évaluer directement, à tout moment et pour toute position relative table-arceau, les dimensions exactes en valeur absolue de ce qui apparaît sur l'image obtenue.

La mesure technique proposée pour cet objet principal est l'étalonnage préalable de l'installation.

A cet effet, l'invention propose d'abord un procédé d'étalonnage d'une installation de radiologie, plus spécialement destinée à des procédures d'angiographie ou d'angioplastie, installation de radiologie comprenant une table de position générale au moins sensiblement horizontale, sur laquelle peut reposer le corps du patient à examiner, déplaçable et blocable ; un arceau notamment isocentrique supportant à ses deux extrémités des moyens d'émission· d'un faisceau de rayons X et des moyen de réception du dit faisceau, déplaçables et blocables ; des moyens de stockage, traitement, visualisation d'images associés aux moyens de réception, notamment par analyse numérique des images produites, grâce à un ordinateur d'analyse des images ; et un ordinateur de réception et de commande des positions de la table et des moyens d'émission et réception reliés à l'ordinateur d'analyse des images ; procédures d'angiographie ou d'angioplastie dans lesquelles on place le corps d'un patient sur la table, on règle les positions relatives de la table et des moyens d'émission et de réception de rayons X (amplificateur), on met en oeuvre les moyens de stockage, traitement, visualisation d'images, caractérisé en ce qu' avant une procédure d'angiographie ou d'angioplastie et en l'absence d'un patient sur la table on étalonne l'installation de manière à connaître ses coefficients d'agrandissement et de distorsion et donc l'échelle des images obtenues, et à cet effet : pour les positions relatives de la table et des moyens d'émission et de réception :

(i) On place la table et les moyens d'émission et de réception (amplificateur), dans une première position relative ;

(ii) Dans cette première position relative, on interpose sur le faisceau de rayons X, une grille calibrée et opaque aux rayons X, dont le centre est aligné avec le centre de l'amplificateur, au moins sensiblement parallèlement au plan de l'amplificateur ; le centre de la grille se trouvant, dans cette première position relative de la table et de l'amplificateur à égale distance entre le plan de la table et le centre de l'amplificateur ;

(iii) Dans cette première position relative et en présence de cette grille, on met en oeuvre les moyens de stockage, traitement, visualisation, un court moment pour obtenir une image de la grille ;

(iv) On compare les dimensions et surfaces de la grille calibrée et de son image afin de connaître, pour cette première position relative, les coefficients d'agrandissement et de distorsion et l'échelle de l'image réalisée ;

(v) On place ensuite la table et les moyens d'émission et de réception (amplificateur) dans une deuxième position relative différente de la première et pas trop éloignée ;

(vi) Dans cette deuxième position relative, on règle, la cas échéant la position de la grille pour l'adapter si nécessaire -notamment on la déplace puis on la bloque en position- et on met également en oeuvre les moyens de stockage, traitement, visualisation, pour connaître, pour cette seconde position, les coefficients d'agrandissement et de distorsion et l'échelle de l'image réalisé ;

(vii) On transmet à l'ordinateur les paramètres caractérisant en positions relatives de la table, des moyens d'émission et de réception (amplificateur) ainsi que les valeurs correspondantes des coefficients d'agrandissement et de distorsion et l'échelle.

(viii) On interpole les coefficients d'agrandissements et de distorsion ainsi que l'échelle pour toute position relative intermédiaire comprise entre la première et la deuxième positions relatives lorsque nécessaire;

(ix) On recommence les étapes précédentes pour d'autres positions relatives et les positions relatives intermédiaires souhaitées ;

(x) On stocke les coefficients d'agrandissement et de distorsion les échelles et les paramètres caractérisant les différentes positions relatives, ainsi que les valeurs interpolées.

L'invention propose ensuite une installation de radiologie, plus spécialement destinée à des procédures d'angiographie ou d'angioplastie, comprenant une table de position générale au moins sensiblement horizontale, sur laquelle peut reposer le corps du patient à examiner, portée des moyens supports de table déplaçables et blocables, grâce à des moyens d'entraînement de table avec blocage et des moyens de commande de la position de la table ; un arceau, notamment isocentrique, supportant à ses deux extrémités des moyens d'émission d'un faisceau de rayons X et des moyens de réception du dit faisceau, portés par des moyens support d'arceau déplaçables et blocables grâce à des moyens d'entraînement d'arceau avec blocage et des moyens de commande de la position de l'arceau ; des moyens de stockage, traitement, visualisation des images associés aux moyens de réception, notamment par analyse numérique, grâce à un ordinateur d'analyse des images; et un ordinateur associé aux moyens de commande de la position de la table et de l'arceau, caractérisé en ce qu'elle comporte également en premier lieu des moyens d'étalonnage destinés à connaître les coefficients d'agrandissement et de distorsion de l'installation ainsi que l'échelle des images obtenues pour les positions relatives de la table et des moyens d'émission et de réception ; et en second lieu, une interface apte à permettre à tout moment d'une part la transmission des valeurs numériques caractérisant toute position relative de la table et des moyens d'émission et de réception (amplificateur) et, d'autre part la commande des déplacements de la table et des moyens d'émission et de réception à partir d'instructions provenant de l'ordinateur.

L'invention sera bien comprise grâce à la description qui suivra en référence aux dessins annexés dans lesquels :

- La figure 1 est une vue purement schématique, en perspective d'une installation de radiologie selon l'invention.

- La figure 2 est un schéma illustrant l'étalonnage selon l'invention.

- La figure 3 est un des schémas possibles d'interface.

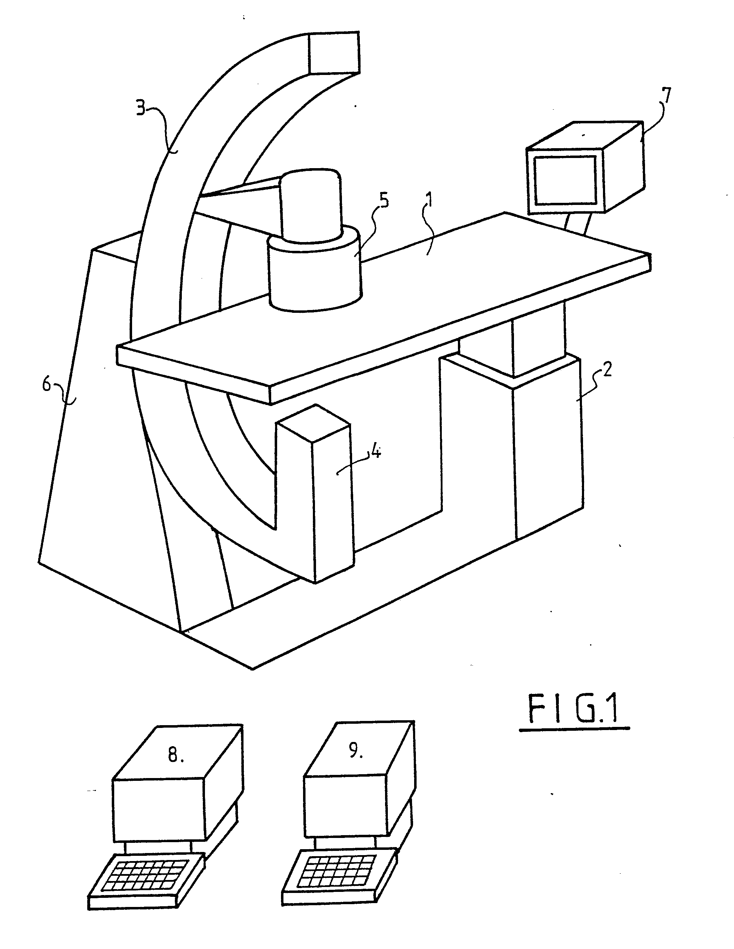

L'invention concerne une installation de radiologie comportant (figure 1) une table 1 de position générale au moins sensiblement horizontale, sur laquelle peut reposer le corps d'un patient à examiner, portée des moyens supports de table 2 déplaçables et blocables, grâce à des moyens d'entraînement de table avec blocage et des moyens de commande de la position de la table ; un arceau 3, notamment isocentrique, supportant à ses deux extrémités des moyens d'émission 4 d'un faisceau de rayons X et des moyens de réception 5 (amplificateur) du dit faisceau, portés par des moyens support d'arceau 6 déplaçables et blocables grâce à des moyens d'entraînement d'arceau avec blocage et des moyens de commande de la position de l'arceau ; des moyens 7 de stockage, traitement, visualisation d'images associés aux moyens de réception, notamment par analyse numérique, grâce à un ordinateur 8 d'analyse d'images; et un ordinateur 9 associé aux moyens de commande de la position de la table et de l'arceau et pouvant être relié à l'ordinateur 8.

Une telle installation ainsi qu'elle vient d'être décrite est parfaitement connue de l'homme du métier et pour cette raison ne nécessite pas d'explications supplémentaires détaillées. Cela est tout spécialement vrai pour les moyens support d'entraînement et de commande de la table et de l'arceau qui ne font pas en soi l'objet de l'invention. Les moyens d'émission 4 sont généralement situés à l'extrémité inférieure de l'arceau 3 et sous la table 1 tandis que les moyens de réception 5 (amplificateur) sont généralement situés à l'extrémité supérieure de l'arceau et au dessus de la table 1 et, en fonctionnement de l'installation, placés directement au contact du malade reposant sur la table 1 et cela dans la zone souhaitée. La table 1, et l'arceau 3 et les moyens d'émission 4 et de réception 5 peuvent être déplacés selon divers mouvements combinables de coulissement et/ou rotation de manière à pouvoir régler la position du faisceau de rayons X par rapport à la table 1, non seulement en ce qui concerne les deux axes (longueur et largeur) de celle-ci -notamment pour un déplacement le long du corps du patient- mais aussi en incidence.

De façon également connue en soi, les moyens de réception 5 (amplificateur) sont généralement placés, en fonctionnement, à proximité immédiate du corps du patient, ainsi que déjà indiqué.

L'ordinateur 8 permettant l'analyse numérique des images et l'ordinateur 9 pour la commande de la table 1 et de l'arceau 3 sont les mêmes ou distincts mais sont reliables fonctionnellement et/ou structurellement l'un à l'autre. Ils comportent naturellement les moyens de commande, l'unité centrale, les périphériques etc..., appropriés et sont chargés avec le ou les programmes convenables.

Les moyens 7 sont représentés sur la figure 1 comme comprenant un ou plusieurs écran(s) de visualisation. Naturellement, ils peuvent comporter d'autres moyens de visualisation temporaires ou permanente ainsi que des moyens de stockage des images obtenues.

Par la suite on désigne par "position relative" de la table 1 et de l'arceau 3, une certaine position définie de la table 1 et des moyens d'émission 4 et de réception 5 (amplificateur), caractérisée par des situations par rapport aux deux axes de la table 1, aux angles d'incidence du faisceau de rayons X par rapport au plan supérieur P de la table 1 (plan de référence) ainsi qu'aux écartements en sens vertical des moyens 4, 5 par rapport à la table 1.

On comprend que ces positions relatives sont en nombre illimité et que pour chacune d'elles il y a une certaine valeur du coefficient d'agrandissement et de distorsion ainsi qu'une certaine échelle.

On entend par échelle, dans le contexte de la présente invention, la distance séparant deux points images sur l'écran des moyens 7 comparativement à la distance réelle entre les deux points origines de l'image. Par exemple le diamètre de l'image d'une artère par rapport au diamètre effectif de l'artère elle-même.

Selon le procédé d'étalonnage suivant l'invention de l'installation de radiologie ainsi définie, avant une procédure d'angiographie ou d'angioplastie et en l'absence d'un patient sur la table 1 on étalonne l'installation de manière à connaître ses coefficients d'agrandissement et de distorsion et donc l'échelle des images obtenues, et, à cet effet : pour les positions relatives de la table 1 et des moyens d'émission et de réception 4, 5:

(i) On place la table 1 et les moyens d'émission et de réception 4, 5, dans une première position relative ;

(ii) Dans cette première position relative, on interpose sur le faisceau de rayons X, une grille 10 calibrée et opaque aux rayons X, dont le centre est aligné avec le centre de l'amplificateur, au moins sensiblement parallèlement au plan de l'amplificateur ; le centre de la grille se trouvant, dans cette première position relative de la table et de l'amplificateur à égale distance entre le plan de la table et le centre de l'amplificateur ;

(iii) Dans cette première position relative et en présence de cette grille 10, on met en oeuvre les moyens d'émission et de réception 4, 5 et les moyens 7 de stockage, traitement, visualisation, un court moment pour obtenir une image de la grille 10;

(iv) On compare les dimensions et surfaces de la grille 10 calibrée et de son image afin de connaître, pour cette première position relative, les coefficients d'agrandissement et de distorsion et l'échelle de l'image réalisée ;

(v) On place ensuite la table 1 et les moyens d'émission et de réception 4, 5 dans une deuxième position relative différente de la première et pas trop éloignée ;

(vi) Dans cette deuxième position relative, on règle la position de la grille 10 pour l'adapter et on met également en oeuvre les moyens d'émission et de réception 4, 5 et les moyens 7 de stockage, traitement, visualisation, pour connaître, pour cette seconde position, les coefficients d'agrandissement et de distorsion et l'échelle de l'image réalisée et cela conformément à la procédure déjà décrite;

(vii) On transmet à l'ordinateur les paramètres caractérisant en positions relatives de la table, des moyens d'émission et de réception (amplificateur) ainsi que les valeurs correspondantes des coefficients d'agrandissement et de distorsion et l'échelle.

(viii) On interpole les coefficients d'agrandissements et de distorsion ainsi que l'échelle pour toute position relative intermédiaire comprise entre la première et la deuxième positions relatives lorsque nécessaire;

(ix) On recommence les étapes précédentes pour d'autres positions relatives et les positions relatives intermédiaires souhaitées ;

(x) On stocke les coefficients d'agrandissement et de distorsion, les échelles et les paramètres caractérisant les différentes positions relatives, ainsi que les valeurs interpolées.

Les positions relatives dans lesquelles sont faites les mesures sont soit prises au hasard, soit correspondent à des positions classiques d'investigation, soit découlent d'une incrémentation des paramètres définissant une position.

L'écart entre deux positions relatives dans lesquelles une mesure est faite est à la portée de l'homme du métier en fonction notamment des moyens de calcul mis en oeuvre pour l'interpolation, de la précision souhaitée pour les calculs des valeurs par interpolation, de l'importance de la variation des coefficients et échelles recherchés par rapport à la variation des paramètres définissant les positions.

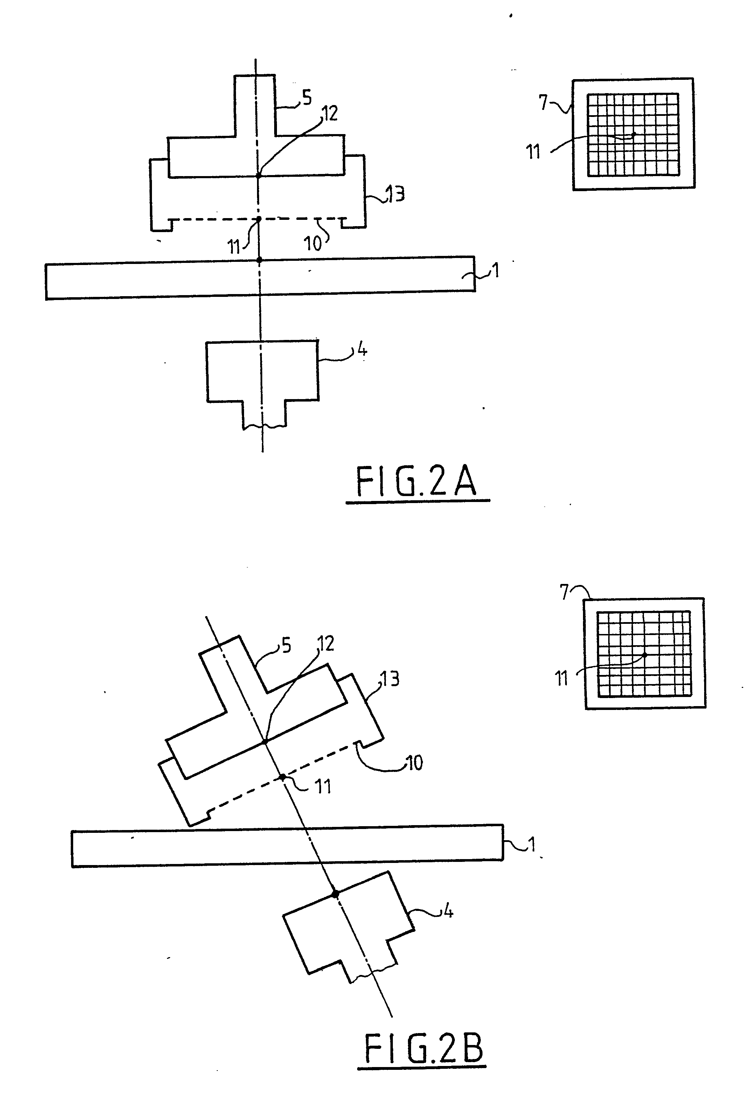

Pour interposer la grille 10, ainsi qu'il est prévu à l'étape (ii), on met en place, au centre de la grille 10 et au centre de l'amplificateur 5, deux billes opaques aux rayons X (en plomb), 11 et 12 respectivement, pour qu'elles soient alignées et superposées, la grille 10 étant portée par une suspente 13 notamment portée par l'amplificateur 5. De cette manière la grille 10 est parallèle au plan de l'amplificateur 5, en permanence. De plus, (voir figure 2B plus spécialement) les écartements entre le centre de la grille et d'une part le plan de l'amplificateur 5 d'autre part le plan P de la table 1 sont égaux.

Une installation selon l'invention est donc caractérisée en ce qu'elle comporte également des moyens d'étalonnage destinés à connaître les coefficients d'agrandissement et de distorsion de l'installation ainsi que l'échelle des images obtenues pour les positions relatives de la table et des moyens d'émission et de réception.

Préférentiellement l'invention met en oeuvre un ordinateur 9 apte à effectuer les interpolations souhaitées ainsi que le stockage des coefficients et échelles pour chaque position. Cet ordinateur 9 est distinct ou non de celui 8 traitant les images et / ou commandant les déplacements relatifs mais il lui est associé.

Il est à noter que l'étalonnage ainsi décrit peut être réalisé originellement avant la première mise en oeuvre de l'installation et ensuite vérifiée à intervalles de temps réguliers afin de s'assurer de la constance des valeurs obtenues ou, le cas échéant, les corriger (notamment en cas de modifications ou de remplacement de la caméra et / ou de son objectif).

L'invention prévoit également que lors de la mise en oeuvre ultérieure de l'installation, après étalonnage, les valeurs de coefficients et échelles soient affichés sur l'écran des moyens 7, lors du fonctionnement et cela pour toute position particulière relative de la table 1 et de l'arceau 3 ainsi que des moyens 4, 5. Cet affichage peut être instantané grâce à l'étalonnage réalisé préalablement, et grâce à l'interface existant entre l'installation radiologique et l'ordinateur 9.

Dans le cas d'une angioplastie, le praticien peut donc, grâce à l'invention, avoir une connaissance immédiate et précise de la dimension réelle d'une sténose ainsi que de portions saines de l'artère comprenant cette sténose. Il peut donc utiliser le ballon approprié, sans risque d'erreur, sans nécessité de tâtonnements et sans délai.

Naturellement l'invention implique qu'à tout moment la position relative table 1-arceau 3 soit connue, ce qui est rendu possible grâce à l'ordinateur 8 lequel est en mesure, à tout instant d'une part de connaitre la position relative table 1 / arceau 3 / . moyens 4/ 5/ ;et d'autre part commander les déplacements relatifs. Généralement, il est prévu d'une part des moyens d'affichage des paramètres définissant une position relative, notamment sur l'écran des moyens 7, et, d'autre part, des moyens de saisie des valeurs souhaitées pour les paramètres définissant une position relative tels qu'un clavier, ou une souris ou un système d'induction vocale de commande ou autre-. Un logiciel approprié de l'ordinateur permet de commander les déplacements convenables de la table 1 et de l'arceau 3 en fonction d'une position relative initiale et de la position relative ainsi définie.

Le cas échéant, il est également prévu des moyens de stockage des paramètres d'une position relative particulière, notamment répétitive.

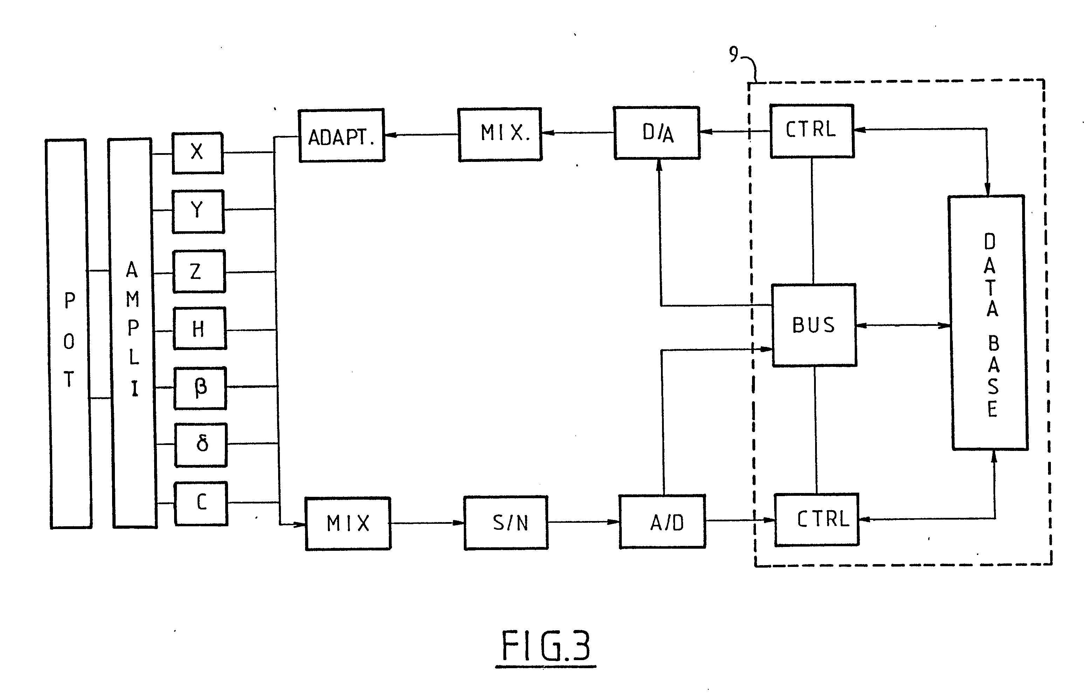

Selon une variante possible de mise en oeuvre de l'invention, les paramètres définissant une position relative table 1-arceau 3 sont les suivants (figure 3) :
- Hauteur table 1 (X)
- Position table 1 antéro postérieure (Y)
- Position table 1 latérale (Z)
- Hauteur moyen réception 5 (H)
- Rotation moyen réception 5 ($\beta$)
- Angulation moyen réception 5 ($\gamma$)
- Champ (C)

Chacun de ces paramètres ainsi que d'autres, éventuels peut varier entre des bornes extrêmes. Les paramètres correspondant à une dimension ou un angle varient de façon continue. Le champ varie généralement de façon discrète, pouvant prendre quelques valeurs (par exemple trois).

Les valeurs des paramètres correspondent à des niveaux réglables de potentiomètres analogiques POT des moyens d'entraînement de la table 1 et de l'arceau 3.

Un circuit approprié permet la transmission à l'ordinateur utilisé pour l'étalonnage des valeurs des paramètres correspondant à la position relative table 1-arceau 3.

Ce circuit peut faire l'object de nombreuses variantes de réalisation. Par exemple si on se réfère expressement à la figue 3 il peut comporter un étage d'amplification AMPLI permettant de normaliser les valeurs de tension de potentiomètres analogiques déjà mentionnés ; un circuit de transmission comportant le mixage des signaux MIX , un échantillonneur/interrupteur S/N ,un convertisseur analogique/digital A/D, aboutissant au bas de l'ordinateur 9 lequel comporte une base de données DATA BASE permettant d'associer à des valeurs spécifiques des paramètres d'une position relative -c'est-à-dire à une position relative déterminée- les valeurs des coefficients d'agrandissement et de distorsion et l'échelle de l'image. Ces valeurs des coefficients et cette échelle sont obtenus, ainsi que décrit, par interpolation à partir de quelques valeurs testées.

Selon une autre caractéristique, l'invention est bien adaptée à une procédure d'angioplastie en temps réel. Initalement, lors de la prise d'un cliché, après injection du produit de contraste, l'opérateur stocke l'image et les paramètres définissant la position relative de l'installation radiologique, et continue son examen en répétant cette procédure un certain nombre de fois. A la fin de l'examen, l'opérateur rappelle les images pour choisir celles correspondant à la meilleure incidence pour effectuer l'angioplastie. Grâce au procédé d'étalonnage selon l'invention, il peut mesurer qualitativement le diamètre de l'artère dans ses portions saines et steosées, par analyse directe de l'image. Enfin, les portions relatives ayant été stockées en même temps que l'image, il peut repositionner automatiquement l'installation dans la position correspondant à la position d'acquisition de l'image.

Selon une autre caractéristique, l' installation est bien adaptée à une procédure d'artérographie des membres inférieurs dans laquelle on effectue une injection dans l'artère suivie d'une modification de la position relative élémentaire elle-même suivie d'un injection et d'une autre modification de la position relative et ainsi de suite. Dans ce cas, il est possible, également, de prévoir un ordinateur de commande des injections (notamment quantativement) en liaison avec l'ordinateur de commande des positions relatives.

Selon une autre caractéristique, le logiciel permettant le changement des positions relatives selon des instructions données par l'opérateur comporte une sécurité consistant à limiter l'amplitude des déplacements selon des bornes correspondant à la morphologie du corps d'un patient sur la table 1, de manière à éviter ainsi toute interférence entre le corps du patient et les moyens de réception 5.

De plus, des capteurs de pression sont prévus sur les moyens de réception 5 de telle manière que lorsqu'ils sont sollicités -ce qui indique une interférence entre les moyens de réception 5 et une partie fixe : table 1 ou corps du patient- les déplacements soient stoppés.

**Revendications**

1. Procédé d'étalonnage d'une installation de radiologie, plus spécialement destinée à des procédures d'angiographie ou d'angioplastie, installation de radiologie comprenant une table (1) de position générale au moins sensiblement horizontale, sur laquelle peut reposer le corps du patient à examiner, déplaçable et blocable ; un arceau (3) notamment isocentrique supportant à ses deux extrémités des moyens d'émission (4) d'un faisceau de rayons X et des moyens de réception (5) du dit faisceau, déplaçables et blocables ; des moyens (7) de stockage, traitement, visualisation d'images associés aux moyens de réception, notamment par analyse numérique, grâce à un ordinateur (8) ; et un ordinateur (9) de commande des positions de la table (1) et des moyens d'émission et réception (4, 5) reliés à l'ordinateur (8) ; procédures d'angiographie ou d'angioplastie dans lesquelles on place le corps d'un patient sur la table (1), on règle les positions relatives de la table (1) et des moyens d'émission et de réception(4, 5) de rayons X, on met en oeuvre les moyens (7) de stockage, traitement, visualisation d'images, caractérisé en ce qu' avant une procédure d'angiographie ou d'angioplastie et en l'absence d'un patient sur la table (1) on étalonne l'installation de manière à connaître ses coefficients d'agrandissement et de distorsion et donc l'échelle des images obtenues, et, à cet effet : pour les positions relatives de la table (1) et des moyens d'émission et de réception (4, 5):

(i) On place la table (1) et les moyens d'émission et de réception (4, 5), dans une première position relative;

(ii) Dans cette première position relative, on interpose sur le faisceau de rayons X, une grille (10) calibrée et opaque aux rayons X, dont le centre est aligné avec le centre de l'amplificateur ; le centre de la grille se trouvant, dans cette première position relative de la table et de l'amplificateur à égale distance entre le plan de la table et le centre de l'amplificateur.

(iii) Dans cette première position relative et en présence de cette grille (10), on met en oeuvre les moyens d'émission et de reception (4,5) et les moyens (7) de stockage, traitement, visualisation, un court moment pour obtenir une image de la grille (10);

(iv) On compare les dimensions et surfaces de la grille (10) calibrée et de son image afin de connaître, pour cette première position relative, les coefficients d'agrandissement et de distorsion et l'échelle de l'image réalisée ;

(v) On place ensuite la table (1) et les moyens d'émission et de réception (4, 5) dans une deuxième position relative différente de la première et pas trop éloignée ;

(vi) Dans cette deuxième position relative on règle la position de la grille (10) pour l'adapter et on met également en oeuvre les moyens d'émission et de reception (4,5) et les moyens (7) de stockage, traitement, visualisation, pour connaître, pour cette seconde position, les coefficients d'agrandissement et de distorsion et l'échelle de l'image réalisée ;

(vii) On transmet à l'ordinateur (9) les paramètres caractérisant en positions relatives de la table, les moyens d'émission et de réception ( amplificateur) (4, 5) ainsi que les valeurs correspondantes des coefficients d'agrandissement et de distorsion et d'échelle.

(viii) On interpole les coefficients d'agrandissements et de distorsion ainsi que l'échelle pour toute position relative intermédiaire comprise entre la première et la deuxième positions relatives losque necessaire ;

(ix) On recommence les étapes précédentes pour d'autres positions relatives et les positions relatives intermédiaires souhaitées ;

(x) On stocke les coefficients d'agrandissement et des distorsion, les échelles, et les paramètres caractérisant les différentes positions relatives, ainsi que les valeurs interpolées.

2. Installation de radiologie, plus spécialement destinée à des procédures d'angiographie ou d'angioplastie, comprenant une table (1) de position générale au moins sensiblement horizontale, sur laquelle peut reposer le corps du patient à examiner, portée des moyens supports de table (2) déplaçables et blocables, grâce à des moyens d'entraînement de table avec blocage et des moyens de commande de la position de la table ; un arceau (3), notamment isocentrique, supportant à ses deux extrémités des moyens d'émission (4) d'un faisceau de rayons X et des moyens de réception (5) du dit faisceau, portés par des moyens support d'arceau (6) déplaçables et blocables grâce à des moyens d'entranement d'arceau avec blocage et des moyens de commande de la position de l'arceau ; des moyens (7) de stockage, traitement, visualisation d'images associés aux moyens de réception, notamment par analyse numérique, grâce à un ordinateur (8) ; et un ordinateur associé aux moyens de commande de la position de la table et de l'arceau, caractérisé en ce qu'elle comporte également des moyens d'étalonnage destinés à connaître les coefficients d'agrandissement et de distorsion de l'installation ainsi que l'échelle des images obtenues pour les positions relatives de la table et des moyens d'émission et de réception et en ce qu'elle permet de connaître à tout moment les paramètres définissant les positions relatives de la table (1), les moyens d'mission (4) et de reception (5).

3. Installation selon la revendication 2, caractérisé en ce que les moyens d'étalonnage comportent une grille calibrée et opaque aux rayons X interposée sur le faisceau de rayons X, sensiblement parallèle aux moyens de reception (5) et dont le centre est à égale distance de ces moyens de réception (5) et de la table (1) ; des moyens pour comparer les dimensions et surfaces de la grille calibrée et de son image et donc obtenir les coefficients d'agrandissement et de distorsion ainsi que l'échelle pour la position relative de la table et les moyens d'émission et de réception ; des moyens d'interpolation des coefficients d'agrandissement et de distorsion ainsi que l'échelle pour toute position relative intermédiaire entre deux positions relatives différentes et pas trop éloignées ; et des moyens permettant de transférer à des moyens de stockage des coefficients d'agrandissement et de distorsion ainsi que des échelles pour les différentes positions relatives et que les paramètres permettant de caractériser la position relative de la table (1) et des moyens d'émission (4) et de réception (5), ce qui permet aussi de commander les positions relatives à partir d'instructions en provenance de l'ordinateur (9).

F I G.1

## FIG.2A

## FIG.2B

FIG.3

EP 0 343 600 A1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | DE-U-8 612 533  (SIEMENS AG)<br>* Page 1, lignes 10-20; page 2, ligne 21 - page 3, ligne 18; page 5, lignes 1-2; figure 1 *<br>--- | 1,2 | A 61 B    6/00 |
| A | DE-A-2 716 818  (VARIAN ASSOCIATES)<br>* Page 20, ligne 24 - page 21, ligne 13; page 21, ligne 30 - page 22, ligne 13; page 31, ligne 29 - page 32, ligne 15; page 33, ligne 28 - page 34, ligne 9; page 36, lignes 1-25; page 37, lignes 10-16; figures 1-5 *<br>--- | 1,2 | |
| A | US-A-3 887 804  (T.J. MORGAN et al.)<br>* Abrégé; colonne 1, lignes 25-45; colonne 2, lignes 16-43; colonne 3, ligne 23 - colonne 4, ligne 47; colonne 6, lignes 1-27; colonne 6, ligne 59 - colonne 7, ligne 29; figures 1-7 *<br>--- | 1-3 | |
| A | WO-A-8 001 111  (KERMATH MFG. CORP.)<br>* Abrégé; page 4, ligne 24 - page 5, ligne 14; page 6, lignes 18-33; page 7, lignes 20-28; page 8, ligne 3 - page 9, ligne 10; figures 1-10 *<br>--- | 1-3 | DOMAINES TECHNIQUES RECHERCHES (Int. Cl.4)<br><br>A 61 B |
| A | FR-A-2 115 423  (VARIAN ASSOCIATES)<br>* Page 4, ligne 36 - page 5, ligne 24; page 5, ligne 36 - page 6, ligne 16; page 7, ligne 40 - page 9, ligne 37; page 14, lignes 4-19; figures 1,2 *<br>----- | 1,2 | |

Le présent rapport a été établi pour toutes les revendications

| Lieu de la recherche | Date d'achèvement de la recherche | Examinateur |
|---|---|---|
| LA HAYE | 17-07-1989 | RIEB K.D. |

CATEGORIE DES DOCUMENTS CITES

X : particulièrement pertinent à lui seul
Y : particulièrement pertinent en combinaison avec un
    autre document de la même catégorie
A : arrière-plan technologique
O : divulgation non-écrite
P : document intercalaire

T : théorie ou principe à la base de l'invention
E : document de brevet antérieur, mais publié à la
    date de dépôt ou après cette date
D : cité dans la demande
L : cité pour d'autres raisons
........................................................................
& : membre de la même famille, document correspondant

EPO FORM 1503 03.82 (P0402)